# EUROPEAN PATENT SPECIFICATION

(11) **EP 2 485 669 B1**
(45) Date of publication and mention of the grant of the patent: **26.08.2015**
(21) Application number: 10773590.4
(22) Date of filing: 05.10.2010
(51) Int. Cl.: A61B 17/80, A61B 17/86

(54) **DEVICE FOR SYNTHESIS OF BONE FRACTURES**
SYNTHESEVORRICHTUNG FÜR KNOCHENBRÜCHE
DISPOSITIF POUR LA RÉDUCTION DE FRACTURES OSSEUSES

(30) Priority: 05.10.2009 SM 200900081
(43) Date of publication of application: 15.08.2012
(73) Proprietor: Lima SM s.p.a., 47899 Serravalle-San Marino (IT)
(72) Inventor: ROTINI, Roberto, I-40136 Bologna (IT); GUERRA, Enrico, I-40068 San Lazzaro Di Savena (IT)
(74) Representative: Petraz, Davide Luigi
(86) International application number: PCT/EP2010/064781
(87) International publication number: WO 2011/042407

(56) References cited:
- EP-A1- 1 764 052
- WO-A1-97/09000
- FR-A1- 2 845 588
- US-A1- 2004 215 195
- US-A1- 2006 235 400
- US-A1- 2008 234 749

## Description

### FIELD OF THE INVENTION

The present invention relates to a device for synthesis of bone fractures.

### BACKGROUND OF THE INVENTION

For synthesis of bone fractures the use of special metal plates is known which are properly shaped to be locked on the outer side of the bone by means of screws. For example, for synthesis of trochanter or femur neck fractures plates, are used which are placed on the fracture edges and fixed by means of screws which are driven with proper inclination in order to penetrate the bone parts which appear to be solid and less injured and therefore able to assure a firm reduction of the fracture.

However the plates generally used present some locking difficulties owing to the fact that the plates do not always permit that the screws are steadily inserted into the fractured segments of the bone with exact and sufficient inclination. In order to permit that the screws assume a greater inclination, it has been suggested to insert an auxiliary bushing or washer between the plate and the screw head having a hemispherical seat to accommodate the complementary head of the screws so as to create an articulation allowing the screws to assume the required inclination. On the other hand the interposition of bushings involves evident economic increase and their use requires an appreciable rise of the plate dimension to get sufficient space for the creation of a hemispherical seat suitable to lodge the screw head. EP 1 764 052 discloses a bone fixation system including a plate and a set of screws. Each locking screw has a head with a self-tapping structure adapted to self-tap into the internal thread of a screw hole of the plate

### SUMMARY OF THE INVENTION

The present invention relates to a device for synthesis of bone fractures as claimed hereafter. Preferred embodiments of the invention are set forth in the dependent claims.

The aim of the present invention is therefore to achieve a device capable to overcome the drawbacks of the known devices and to be economically profitable compared with the traditional ones.

This aim is achieved with a device for synthesis of bone fractures, comprising a plate and screws for locking said plate. Said plate is made from biocompatible and radiolucent plastic material suitable to permit a radiographic control and an easy explantation of the plate and is provided with through holes for the screws fixing the plate on the bone. Said screws comprise a threaded shank having an external diameter less than the diameter of said holes and a head having a thread with an external diameter greater than the diameter of said holes so as to permit insertion of the screws according to a plurality of directions and inclination with respect to the axis of the holes in the plate, said head being suitable to engage said holes by self-threading.

According to some embodiments of the present invention, the holes of the plate are substantially cylindrical and have a diameter such as to permit angular selection of the screw shank relative to the plate. Moreover, in some embodiments, the head is substantially cylindrical and has a helical thread with spiral rings having an external diameter greater than the diameter of the holes so that, when the head is screwed in the holes, the spiral rings engage the hole wall by self-threading so as to lock the screw in the selected angulation.

### BRIEF DESCRIPTION OF THE DRAWINGS

Further characteristics and advantages of the present invention will become more apparent from the following description of a preferred but not exclusive embodiment, illustrated by way of example in the accompanying drawings wherein:
- figure 1 shows a perspective view of a plate according to the invention;
- figure 2 is an enlarged view of a portion of the plate of figure 1;
- figure 3 is a view of a screw partially screwed through the plate;
- figure 4 is a view of a screw entirely screwed through the plate of figure 1.

### DETAILED DESCRIPTION OF A PREFERENTIAL FORM OF EMBODIMENT

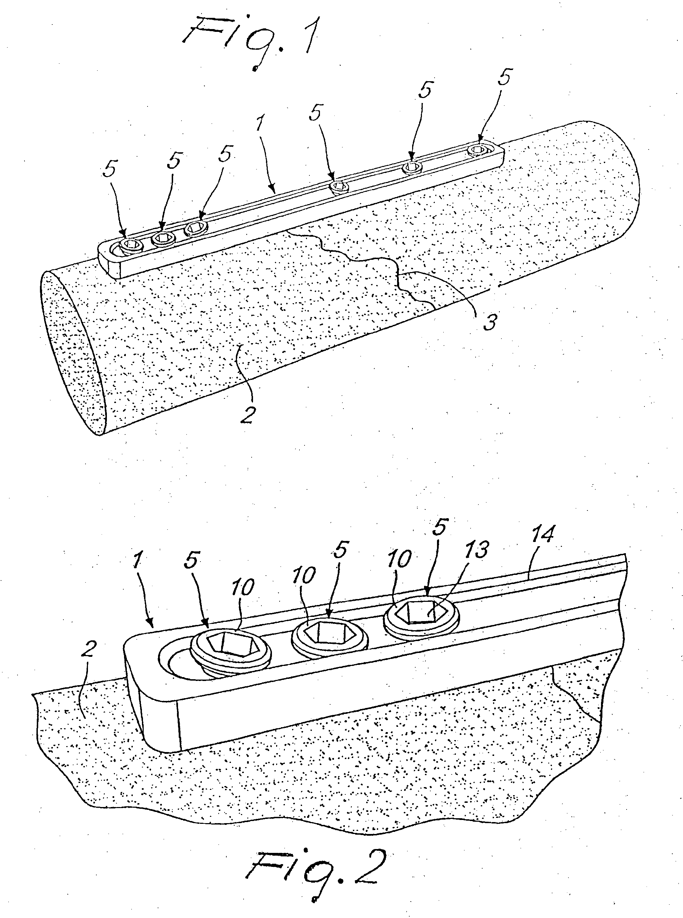

In figures 1 and 2 the reference 1 designates a plate according to the invention comprising a bar entirely made from a biocompatible plastic material POLYETHRETHERKETONE, well known by name of PEEK and suitable to facilitate the explantation of the plate. Preferably this biocompatible plastic material is radiolucent to make the radiocontrol easy and advantageously strengthened by means of carbon fibres.

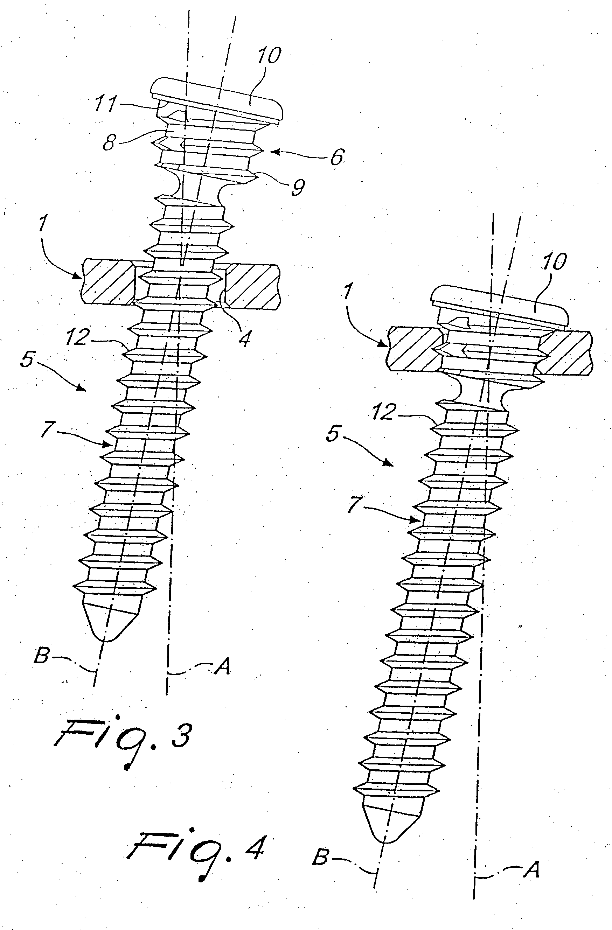

The shape of the plate may be any according to the shape of the bone near to the fracture to be reduced. In figure 1, references 2 and 3 indicate the bone and the fracture edges respectively. In plate 1 a plurality of through holes is formed (see figure 4) the number and disposition of which along the plate depend from the design of the plate and from the anatomical zones in which the use is intended. The holes 4 are preferably cylindrical and their diameter depends from the size of screws 5 which are driven through the holes 4 to lock the plate 1 on the bone 2 and to join the bone portions that compose the fracture 3.

The screws 5 comprise a head 6 and a shank 7. The head 6 comprises a core 8, around which a helical thread 9 extends helically having one or two starts according to the screw type, whereby the spiral rings of the threads have a substantially triangular or orthopaedic section and an external diameter which is greater than the diameter of the hole 4. In some embodiments, the head 6 is cylindrical in shape. In some particular embodiments, the core 8 is cylindrical and has a diameter smaller than that of the hole 4 so that, when the head 6 is screwed in the hole 4, the thread 9 engages the wall of the hole 4. Likewise the core 8 may present a conical shape.

The head 6 at the end opposite to the shank 7, is provided with a collar 10 having a diameter greater than the external diameter of the thread 9, which collar 10 acts as arrest on the upper face of the plate 1 when the head 6 is completely threaded in the hole 4 as it will appear in the following. Advantageously, in order to render the screw 5 more secure against unscrewing once it is tightened, the lower face of the collar 10 comprises a indentation 11 which engages the plate 1 thus preventing unscrewing of the screw 5 in case of bone failure.

The shank 7 comprises a thread 12, for example having spiral rings with substantial triangular or usual in orthopaedic threads and an external diameter smaller than that of the core 8 and therefore such as to render the shank 7 loose when driven through the hole 4. The difference between the diameter of the hole 4 of the plate 1 and the external diameter of the thread of the shank 7 is preferably such as to permit the latter to reach an inclination of at least 15° with respect to the axis A of the hole 6.

The structure of the screws 5 is completed by a recess 13 formed in the head 6 (see figure 4) and having a polygonal shape to permit engagement of an allen spanner by means of which the user may carry out screwing of the screws.

From the above description it will appear evident that the plate 1 may be positioned with high simplicity. Indeed after the plate 1 has been positioned on the bone 2, the screws 5 are applied and the axis B may be arranged with respect to axis A of the holes, according to the inclination necessary to drive the screws into the various areas of the bone 2 which are in condition to give a sufficiently firm grip able to consent reduction of the fracture 3. In a first phase when the plate 1 has been positioned on the bone 2, by means of an allen spanner engaging the recess 13, the screw 5 is screwed until the thread 9 of the head 6 abuts the plate 1. At this moment, a further screwing of the screw 5 causes abutment of the plate 1 on the bone 2 owing to the pressure of the first spiral ring of the thread 9 of the head 6 on the plate 1. When the pressure exerted by the thread 9 on the plate 1 is over a certain value, forced penetration of the thread 9 into the hole 4 begins and the collar 10, acting on the plate 1, causes the definitive tightening on the bone 2.

It will be observed that while locking of the plate 1 on the bone 2 is resulting from a first traction effect exerted by the thread 12 of the shank 7, screwing of the thread 9 gives as result to set at zero the clearance of the screw 5 in the hole 4 and therefore the even if very small displacement that the plate 1 could do and compromise the firmness of the plate 1 and of the fracture 3.

In fact, in order to permit the inclination of the screws 5 according to the necessity required from the appearance of the fracture 3, it would be necessary to provide increase of the hole dimension. However the increase would cause excessive clearance which will have as consequence a locking inadequate and suitable to induce displacement of the plate 1.

On the contrary the present invention suggests a solution with the substantial advantage that the head 6, owing to a thread 9 having a greater diameter, after being forced into the hole 4, permits to nullify the clearance of the increased holes 4 and consequently to firmly lock the plate 1. Advantageously, in order to create a compression effect between plate 1 and bone 2, the threads 9, 12 have a small pitch difference.

Moreover, with the present invention the selection of angulation and locking of the screws is not limited as in the prior art. In fact, the plastic material, e.g. PEEK, of the plate permits the threads of the screws to penetrate by self-drilling the inner wall of the hole also in case of relevant angulation with respect to the plane thus achieving solid anchoring of the screws to the plate.

Thus the described device achieves the intended aims. The use of radiolucent plastic material, unlike to a radio-opaque material (stainless steel, titanium alloy of common use) permits a more efficacious radiographic control of the fracture both in operating field, including the possibility to improve the implantation of the plate, and during periodical post operative controls in order to verify the progress of the recovery of the fracture. Another very important aspect of the invention is the possibility to easily explant the plate 1, contrary to the difficulties found in the explantation of metal plates having threaded holes. In fact in these plates the head of the screws engages the threaded hole of the plate and the unscrewing of the screws during explantation is laborious because of the threads which get stuck between the screw head and the plate.

Numerous modifications and variations are possible in the practical embodiment of the invention. Advantageously the plate 1 is provided with a perimetric stiffening rib 14 (see figures 1, 2). Instead of one rib it is possible to provide few ribs arranged so as to stiff the zones of the plate which are subjected to the greatest efforts.

## Claims

1. Device for synthesis of bone (2) fractures (3), comprising a plate (1) and screws (5) for locking said plate (1), said plate (1) being provided with through holes (4) for the screws (5) fixing the plate (1) on the bone (2) and said screws (5) comprising a threaded shank (7) having an external diameter less than the diameter of said holes (4) and a head (6) suitable to engage said holes (4) by self-threading, said head (6) having a helical thread (9) with spiral rings having an external diameter greater than the diameter of said holes (4) so that, when the head (6) is screwed in the holes (4), the spiral rings engage the hole wall by self-threading so as to lock the screw (5) in the selected angulation, so as to permit insertion of the screws (5) according to a plurality of directions arid inclination with respect to the axis of the holes (4) in the plate (1), wherein said holes (4) of the plate (1) are substantially cylindrical and have a diameter such as to permit angular selection of the screw shank (7) relative to the plate (1), **characterized in that** said plate (1) is entirely made from biocompatible and radiolucent plastic material suitable to permit a radiographic control and an easy explantation of the plate (1), **and in that** said head (6) is substantially cylindrical.

2. Device according to claim 1, **characterized in that** said radiolucent material is suitable to permit a radiographic control during reduction of the fracture.

3. Device according to one of the above claims 1 or 2, **characterized in that** said head (6), at the end opposite to the shank (7), is provided with a collar (10) having a diameter greater than the external diameter of the threading (9) of said head and suitable to act as abutment on the upper face of the plate when the head (6) is completely screwed in said hole (4).

4. Device according to claim 3, **characterized in that** said collar (10) is provided with an indentation (11) on the face facing said threading (9).

5. Device according to one of the above claims, **characterized in that** said biocompatible and radiolucent plastic material is POLYETHERETHER-KETONE known on the market as PEEK.

6. Device according to claim 5, **characterized in that** said biocompatible and radiolucent plastic material is POLYETHERETHER-KETONE known on the market as PEEK and stiffened with carbon fibres.

7. Device according to one of the above claims, **characterized in that** said biocompatible and radiolucent plastic material is suitable to aid explantation of the head (6) of the screws from the holes (4) of said plate (1).

8. Device according to one of the above claims, **characterized in that** said plate (1) is provided with stiffening ribs.

9. Device according to claim 8, **characterized in that** said plate (1) is provided with a perimetric stiffening rib (14).

## Patentansprüche

1. Vorrichtung zur Synthese von Brüchen (3) von Knochen (2), umfassend eine Platte (1) und Schrauben (5) zum Verriegeln der Platte (1), wobei die Platte (1) mit Durchgangslöchern (4) für die Schrauben (5) versehen ist, die die Platte (1) am Knochen (2) fixieren, und die Schrauben (5) einen Gewindeschaft (7) mit einem Außendurchmesser kleiner als der Durchmesser der Löcher (4) und einen Kopf (6) aufweisen, der zum Eingriff in die Löcher (4) durch Selbstschrauben geeignet ist, wobei der Kopf (6) ein Schraubengewinde (9) mit spiralförmigen Ringen mit einem Außendurchmesser größer als der Durchmesser der Löcher (4) aufweist, so dass, wenn der Kopf (6) in die Löcher (4) geschraubt wird, die spiralförmigen Ringe durch Selbstschrauben in die Lochwand eingreifen, um die Schraube (5) unter der gewählten Winkelbildung zu verriegeln, um ein Einführen der Schrauben (5) gemäß mehreren Richtungen und einer Neigung in Bezug auf die Achse der Löcher (4) in der Platte (1) zu ermöglichen, wobei die Löcher (4) der Platte (1) im Wesentlichen zylindrisch sind und einen solchen Durchmesser aufweisen, dass sie eine Winkelauswahl des Schraubenschafts (7) relativ zur Platte (1) ermöglichen, **dadurch gekennzeichnet, dass** die Platte (1) vollständig aus einem bioverträglichen und strahlendurchlässigen Kunststoffmaterial gefertigt ist, das geeignet ist, eine Röntgenuntersuchung und eine einfache Explantation der Platte (1) zu erlauben, und dass der Kopf (6) im Wesentlichen zylindrisch ist.

2. Vorrichtung nach Anspruch 1, **dadurch gekennzeichnet, dass** das strahlendurchlässige Material geeignet ist, eine Röntgenuntersuchung während der Reposition des Bruchs zu erlauben.

3. Vorrichtung nach einem der vorstehenden Ansprüche 1 oder 2, **dadurch gekennzeichnet, dass** der Kopf (6) am Ende gegenüber dem Schaft (7) mit einem Kragen (10) versehen ist, der einen Durchmesser aufweist, welcher größer ist als der Außendurchmesser des Gewindes (9) des Kopfes und dafür geeignet ist, als Widerlager auf der oberen Fläche der Platte zu dienen, wenn der Kopf (6) vollständig in das Loch (4) eingeschraubt ist.

4. Vorrichtung nach Anspruch 3, **dadurch gekennzeichnet, dass** der Kragen (10) mit einer Vertiefung (11) auf der Seite, die zum Gewinde (9) weist, versehen ist.

5. Vorrichtung nach einem der vorstehenden Ansprüche, **dadurch gekennzeichnet, dass** das bioverträgliche und strahlendurchlässige Kunststoffmaterial auf dem Markt als PEEK bekanntes POLYETHERETHERKETON ist.

6. Vorrichtung nach Anspruch 5, **dadurch gekennzeichnet, dass** das bioverträgliche und strahlendurchlässige Kunststoffmaterial das als PEEK auf dem Markt bekannte POLYETHERETHERKETON ist und mit Kohlefasern versteift ist.

7. Vorrichtung nach einem der vorstehenden Ansprüche, **dadurch gekennzeichnet, dass** das bioverträgliche und strahlendurchlässige Kunststoffmaterial geeignet ist, die Explantation des Kopfes (6) der Schrauben aus den Löchern (4) der Platte (1) zu unterstützen.

8. Vorrichtung nach einem der vorstehenden Ansprüche, **dadurch gekennzeichnet, dass** die Platte (1) mit Versteifungsrippen versehen ist.

9. Vorrichtung nach Anspruch 8, **dadurch gekennzeichnet, dass** die Platte (1) mit einer perimetrischen Versteifungsrippe (14) versehen ist.

## Revendications

1. Dispositif pour la synthèse de fractures (3) d'os (2), comportant une plaque (1) et des vis (5) pour bloquer ladite plaque (1), ladite plaque (1) étant pourvue de trous traversants (4) pour les vis (5) fixant la plaque (1) sur l'os (2) et lesdites vis (5) comportant une tige filetée (7) ayant un diamètre extérieur inférieur au diamètre desdits trous (4) et une tête (6) adaptée pour s'engager dans lesdits trous (4) par auto-filetage, ladite tête (6) ayant un filet hélicoïdal (9) avec des anneaux en spirale ayant un diamètre extérieur supérieur au diamètre desdits trous (4) de sorte que, lorsque la tête (6) est vissée dans les trous (4), les anneaux en spirale s'engagent dans la paroi de trou par auto-filetage de manière à bloquer la vis (5) dans l'angulation sélectionnée, de manière à permettre l'insertion des vis (5) suivant une pluralité de directions et une inclinaison par rapport à l'axe des trous (4) dans la plaque (1), dans lequel lesdits trous (4) de la plaque (1) sont sensiblement cylindriques et ont un diamètre tel qu'ils permettent une sélection angulaire de la tige filetée (7) par rapport à la plaque (1), **caractérisé en ce que** ladite plaque (1) est entièrement constituée d'une matière plastique biocompatible et radiotransparente, adaptée pour permettre un contrôle radiographique et une explantation aisée de la plaque (1), et **en ce que** ladite tête (6) est sensiblement cylindrique.

2. Dispositif selon la revendication 1, **caractérisé en ce que** ladite matière radiotransparente est adaptée pour permettre un contrôle radiographique pendant une réduction de la fracture.

3. Dispositif selon l'une des revendications 1 ou 2 ci-dessus, **caractérisé en ce que** ladite tête (6), sur l'extrémité opposée à la tige (7), est pourvue d'une couronne (10) ayant un diamètre supérieur au diamètre externe du filetage (9) de ladite tête et adaptée pour agir comme une butée sur la face supérieure de la plaque lorsque la tête (6) est entièrement vissée dans ledit trou (4).

4. Dispositif selon la revendication 3, **caractérisé en ce que** ladite couronne (10) est pourvue d'une indentation (11) sur la face dirigée vers ledit filetage (9).

5. Dispositif selon l'une des revendications ci-dessus, **caractérisé en ce que** ladite matière plastique biocompatible et radiotransparente est une POLYÉTHERÉTHER-CÉTONE appelée PEEK sur le marché.

6. Dispositif selon la revendication 5, **caractérisé en ce que** ladite matière plastique biocompatible et radiotransparente est une POLYÉTHERÉTHER-CÉTONE appelée PEEK sur le marché, et rigidifiée avec des fibres de carbone.

7. Dispositif selon l'une des revendications ci-dessus, **caractérisé en ce que** ladite matière plastique biocompatible et radiotransparente est adaptée pour aider à l'explantation de la tête (6) des vis à partir des trous (4) de ladite plaque (1).

8. Dispositif selon l'une des revendications ci-dessus, **caractérisé en ce que** ladite plaque (1) est pourvue de nervures de raidissement.

9. Dispositif selon la revendication 8, **caractérisé en ce que** ladite plaque (1) est pourvue d'une nervure de raidissement périmétrique (14).
